(19) Europäisches Patentamt
European Patent Office
Office européen des brevets

(11) EP 1 609 455 A1

# (12) EUROPEAN PATENT APPLICATION

(43) Date of publication:
**28.12.2005 Bulletin 2005/52**

(51) Int Cl.[7]: **A61K 7/13**, A61K 7/135

(21) Application number: **05011328.1**

(22) Date of filing: **25.05.2005**

(84) Designated Contracting States:
**AT BE BG CH CY CZ DE DK EE ES FI FR GB GR HU IE IS IT LI LT LU MC NL PL PT RO SE SI SK TR**
Designated Extension States:
**AL BA HR LV MK YU**

(30) Priority: **24.06.2004 EP 04253769**

(71) Applicant: **THE PROCTER & GAMBLE COMPANY
Cincinnati, Ohio 45202 (US)**

(72) Inventors:
- **Glenn, Robert Wayne
Surrey, GU 15 3DL (GB)**
- **James, Delyth Anghared
Egham, Surrey, TW20 8HU (GB)**

(74) Representative: **Kohol, Sonia et al
Procter & Gamble Technical Centres Limited
Patent Department
Rusham Park,
Whitehall Lane
Egham, Surrey TW20 9NW (GB)**

## (54) Hair colouring and bleaching compositions

(57) This invention is a two part system for coloring or bleaching hair which comprises (i) a thin lotion optionally comprising an oxidative dye and comprising a solvent system and a surfactant system to trigger a thickening upon dilution with a developer, and (ii) a developer which comprises a scalp-irritating amount of an oxidising agent. In the system of the invention, the lotion has a Lamellar Phase Index of greater than 80%.

EP 1 609 455 A1

Printed by Jouve, 75001 PARIS (FR)

## Description

FIELD OF THE INVENTION

The present invention relates to two part aqueous compositions for colouring or bleaching hair. In particular, it relates to the reduction of sensory irritation caused by the presence of an oxidizing agent in certain hair coloring or hair bleaching compositions.

BACKGROUND OF THE INVENTION

Oxidative dyeing and bleaching of hair usually involves the use of a two part system. One part is the lotion, also called tint, which contains a variety of ingredients, including the oxidation dye precursors, high pH buffer and rheology modifiers, and the second part the developer which contains a suitable stabilised oxidising agent. Upon mixing of the lotion and developer the bleaching/oxidative dyeing chemistry is initiated. For consumer convenience typically the two compositions are formulated as thin liquids for ease of mixing, once mixed they form a thickened liquid solution for example a lotion, cream or gel.

It is well known that compositions for coloring or bleaching hair comprise oxidizing agents such as hydrogen peroxide. The oxidant not only serves to oxidise the primary dye intermediates but also to destroy some of the natural melanin pigment in the hair. It is known that the efficacy of these compositions depends to some extent on the amount of oxidizing agent that is present. In use, it has been found that developers which have a higher content of oxidizing agent, when mixed with thin lotions, will cause a perceived sensory irritation of the user's scalp that can be described as stinging, tingling, itching, or burning or descriptors there like. If the amount of oxidizing agent is reduced, the intensity or the sensory irritation reduces or disappears altogether, however, the efficacy of the composition decreases. There is therefore a need to formulate thin, hair coloring or bleaching lotions, which will cause little or no sensory irritation when mixed with a developer containing scalp irritating amounts of an oxidizing agent.

Several attributes are desirable in procedures using oxidative dyes to color hair:

- The lotions must be stable to insure a reasonable shelf life
- The composition formed by mixing the lotion and developer must have rheological properties such that it can readily be distributed throughout the hair mass when applied by use of a brush or with fingers, yet in the absence of stress dripping or running from the hair during the colour development period can be substantially avoided. The composition must also be readily rinseable from the hair with water.
- The composition formed by mixing the lotion and developer, as applied to hair, should allow rapid diffusion of the dye precursors from the dye mixture into the hair fibre.
- The lotion and developer should preferably, but not necessarily, have comparable viscosities in order to facilitate mixing.

The major thickening mechanism in commercial hair colorants is due to amphiphilic molecules which form aggregates in aqueous solution. The formation and type of these aggregates is dependent upon the amphiphile molecular structures, their concentration and the nature of the surrounding environment. The most common form of amphiphile aggregates formed within hair colorants are either worm-like micelles or lamellar phases [See Polish Journal of Pharmacology, 1999, 51, 211-222 ISSN 1230-6002 for descriptions]. The former worm-like micelle thickened hair colorants result in the formation of an isotropic and viscous gel for application to hair, whereas the latter lamellar phase thickened hair colorants result in the formation of an anisotropic and viscous cream/gel for application to the hair. For both cases, the thickening is suppressed in the original bottles by employing the amphiphiles in the tint lotion formulation together with a high level of solvents, (typically greater than 20 wt. %) which is added by the consumer to a predominantly aqueous developer bottle to initiate the thickening (dilution thickening) in-situ immediately prior to application to hair.

It has now been found that hair colorants or bleaches that rely on worm-like micelle thickening are less ideal for skin sensory irritation versus lamellar phase thickened hair colorants. Given that the lamellar liquid crystal aggregates are insoluble, their presence can be determined by measurement via turbidimetry. To compare and contrast hair colorant or bleaching formulations, a turbidity methodology was developed wherein the tint lotion formulations (which contain the "thickening" amphiphiles in combination with high levels of solvents to suppress thickening in the bottle) is diluted with de-ionized water with turbidity values measured as a function of the amount of water added, so as to mimic dilution via the developer formulations. The formulae displaying a significant increase in turbidity values with added

water are lamellar phase thickened hair colorants (opaque creams/gels), whereas the formulas which do not display such an increase in turbidity are worm-like micelle thickened colorants (clear gels). For lamellar phase thickened hair colorants or bleaches, the amount of water required to reach a turbidity value of 100 (wt.% of water added of the total mixture) is measured and subtracted from 100% to compute the Lamellar Phase Index value (lamellar phase index = 100% - wt.% of added water for turbidity of 100). This methodology is described further below.

Importantly, by using this turbidity methodology it was surprisingly discovered that lamellar phase thickening hair lotions with Lamellar Phase Indexes of greater than 80% cause less sensory irritation than lotions with Lamellar Phase Indexes of less than 80%. Moreover, it was also found that all hair colorant tint formulations which are commercially available and which were tested had a Lamellar Phase Index of less than 80%. Importantly, however, a lotion with an Index of 100% is not found to be stable. Accordingly, the compositions herein have a Lamellar Phase Index of from 80% to less than 100%, preferably from 80% and 95%, more preferably from 82% to 92%, and most preferably between 85% and 90%. To achieve such lotions, it has also been discovered that it is not only the absolute level of solvent employed that is important, but also the type of solvent that is employed. Surprisingly, it has been discovered that more hydrophilic solvent systems having a lower weighted average ClogP value as described below offer improved sensory irritation versus more hydrophobic solvents with higher weighted average ClogP values. The solvent systems of the present invention have a weighted average ClogP of less than 0.0, preferably of less than -0.1 and more preferably of less than -0.3

Additionally, it has been discovered that hair colorant or bleaching lotions in the present invention preferably have a required range of mono-ethanolamine (MEA) within the formula. Accordingly, preferred hair colorant or bleaching lotions of the present invention comprise less than 3.5 wt. % MEA, preferably less than 3 wt. % MEA, and more preferably less than 1% MEA, or from 6 wt % to 20 wt %, more preferably 9 wt % to 15 wt %MEA.

## SUMMARY OF THE INVENTION

In one embodiment, the invention is a two part system for coloring or bleaching hair which comprises (i) a thin lotion optionally comprising a dye and comprising a solvent system and a surfactant system to trigger a thickening upon dilution with a developer, and (ii) a developer which comprises a scalp-irritating amount of an oxidising agent, characterised in that said lotion has a Lamellar Phase Index of greater than 80% and less than 100%.

In a preferred embodiment, the lotion in the system of the invention comprises a solvent system with a weighted average ClogP of less than 0.0, and the lotion comprises less than 3 % by weight of monoethanolamine (MEA).

In another embodiment, the invention comprises a process of using the system above,
wherein said lotion and said developer are mixed together so as to create a thickened coloring or bleaching composition, and said thickened coloring or bleaching composition is applied to hair, left to act, then rinsed off.

## DETAILED DESCRIPTION OF THE INVENTION

This invention is a two part system for coloring hair which comprises (i) a lotion and (ii) a developer.

### <u>1 - The Lotion:</u>

The lotion comprises a solvent system, a surfactant system and optionally an oxidative dye precursor, and the lotion has a Lamellar Phase Index value of greater than 80% and less than 100%. The lotion herein is thin, i.e. it has a viscosity of 1 cps to 2000 cps, preferably 1 cps to 500 cps (all viscosities herein are measured at 6 rpm with a Brookfield DV-11+ viscometer with a T-Bar D spindle at 25°C).

### Lamellar Phase Index Determination

The Lamellar Phase Index of hair colorant tint formulations is determined measuring the turbidity values as a function of added de-ionized water via an LP2000 Turbidity Meter from Hanna Instruments, Bedfordshire, United Kingdom. For lamellar phase thickened hair colorants, the amount of water required to reach a turbidity value of 100 (wt. % of water added of the total mixture) is measured. This value is then subtracted from 100% to compute the lamellar phase index value (= 100% - wt. % of water to give turbidity of 100). The turbidity meter is calibrated before use as detailed in instruction manual. All measurements were taken at 25°C. The measurement protocol is as follows: A 50ml beaker is thoroughly cleaned, including prior rinsing with ethanol, and then dried:

1. 20 grams (+/- 0.0015g) of the thin hair colorant tint is weighed directly into the beaker.
2. Deionised water is then added dropwise to the tint and then stirred at regular intervals with a glass rod for 20 seconds with special attention that the tint is completely removed from the bottom of the beaker and thereby mixed

properly.

Suggested intervals for stirring are after addition of 0.15grams (+/-0.0500g) of deionised water. Aeration of the tint should be avoided with the use of a sonicator bath to remove any bubbles formed as may be necessary.

3. Immediately dispense 10ml (to line in cuvette) of resulting liquid into a clean cuvette by pouring liquid down the inside of the cuvette (Note: The cuvette is thoroughly rinsed with ethanol and then dried prior to readings) which is then loaded into the turbidity meter.

4. After approximately 30 seconds a turbidity reading is recorded, immediately followed by a second and third reading for verification.

## 1.1 - The Surfactant System:

The lotion herein comprises from 2 % to 30% by weight of the total composition of a surfactant system, preferably from 3% to 15%, most preferably 3.5% to 10%. The surfactant system for use herein has the main function of creating - together with the solvent - a system which thickens upon dilution with the developer. As discussed hereinabove, the surfactant system must provide a lamellar phase index value of greater than 80% within the lotion formulation environment, in order to reduce or eliminate sensory irritation.

It is well known that in aqueous solutions, certain amphiphilic molecules and combinations form lamellar liquid crystals (neat phase) which are mesomorphic structure types between liquids, which are random, and crystals, which are periodic in three dimension. [See F. B. Rosevear, "Liquid Crystals: The Mesomorphic Phases of Surfactant Compositions", J. Soc. Cosmetic Chemists, 19, 581-594 (Aug. 19, 1968)]. Such lamellar liquid crystals consist of parallel bimolecular layers, termed bi-layers, swollen in water wherein the hydrophilic polar head groups orient toward the aqueous environment and the hydrophobic tail regions orient toward the center where they are shielded from the aqueous environments. Parallel sheets of water separate successive bi-layers to create a multi-bi-layered system. Such bi-layers can be present dispersed in the form of vesicles (also termed liposomes) wherein the bi-layers adopt a spherical configuration to surround an aqueous space. Liposomes consisting of a single bi-layer are referred to as unilamellar vesicles (ULV), and those consisting of two or more bi-layers (onion-like structures) as multilamellar vesicles (MLV). ULV's can be small (<100 nm in diameter) or large (> 100 nm). MLV's can be 100 nm to > 1 mm in diameter.

The dilution thickening lamellar phase bi-layers of the present invention can be in the form of flat sheets, unilamellar vesicles and multilamellar vesicles or combinations thereof.

Many amphiphiles, or surfactants, are known to form bi-layers depending on the degree of packing of adjacent surfactants within aqueous solution. This degree of packing is influenced by surfactant structure as well as packing synergisms that may exist between differing surfactants within mixed surfactant systems. As a general rule of thumb, the Critical Packing Factor ($\underline{\phi}$) can be utilized to visualize the surfactant structural characteristics that influence the degree of packing:

$$\underline{\phi}=v/(a x L)$$

where v is the volume occupied by hydrophobic groups within the core, a is the cross-sectional area occupied by the hydrophilic groups at the surface, and L is the length of the hydrophobic groups. For instance, as the hydrophobic group becomes bulkier, the packing will be increased. Also, it can be visualized that the addition of co-surfactants such as fatty alcohols (small a and large v and L) will also result in increased packing. While not being bound to theory, it is generally believed that the packing parameter ($\underline{\phi}$) needs to be greater than 0.5 and less than or equal to 1.0 for bi-layers to be formed. If $\underline{\phi} < 0.5$ the formation of micelles will be favorable and if $\underline{\phi} > 1.0$ the formation of reverse micelles will be favourable.

Exemplary classes of surfactants capable of participating in the formation of a lamellar liquid crystal structure include, but are not limited to specific cationic surfactants, anionic surfactants, nonionic surfactants, quaternary ammonium surfactants and lipid surfactants.

Specific nonionic surfactants are fatty alcohols or fatty acids, or derivatives thereof, or a mixture of any of these, having a chain length of from about 14 to about 20 carbon atoms. These materials may be predominantly linear or may be branched and can be saturated or partially unsaturated. Some examples include myristyl alcohol, myristic acid, cetyl alcohol, palmitic acid, cetearyl alcohol, stearyl alcohol, stearic acid, oleic acid, oleyl alcohol, arachidyl alcohol, arachidic acid, and mixtures thereof.

Other specific non-ionic surfactants include condensation products of C14 to C22 aliphatic primary or secondary linear or branched and saturated or unsaturated chain alcohols or phenols with alkylene oxides, usually ethylene oxide, and generally having from 1 to 50 ethylene oxide groups. Some examples include, but are not limited to, ceteth-1, ceteth-2, ceteth-3, ceteth-4, ceteth-5, ceteth-6, ceteth-10, ceteth-12, ceteth-14, ceteth-15, ceteth-16, ceteth-20, ceteth-24, ceteth-25, ceteth-30, ceteareth-2, ceteareth-3, ceteareth-4, ceteareth-5, ceteareth-6, ceteareth-7, ceteareth-8, ceteareth-9, ceteareth-10, ceteareth-11, ceteareth-12, ceteareth-13, ceteareth-14, ceteareth-15, ceteareth-16, ceteareth-17, ceteareth-18, ceteareth-20, ceteareth-22, ceteareth-23, ceteareth-24, ceteareth-25, ceteareth-27, ceteareth-28, ceteareth-29, ceteareth-30, steareth-2, steareth-3, steareth-4, steareth-5, steareth-6, steareth-7, steareth-8, steareth-10, steareth-11, steareth-13, steareth-14, steareth-15, steareth-16, steareth-20, steareth-21, steareth-25, steareth-27, steareth-30, arachideth-20, beheneth-5, beheneth-10, beheneth-20, beheneth-25, beheneth-30, oleth-2, oleth-3, oleth-4, oleth-5, oleth-6, oleth-7, oleth-8, oleth-9, oleth-10, oleth-11, oleth-12, oleth-15, oleth-16, oleth-20, oleth-23, oleth-25, oleth-30, oleth-40, oleth-44, oleth-50, pareth-3, pareth-9, pareth-12, and mixtures thereof.

Specific cationic surfactants include quaternary ammonium halides, e.g., alkyltrimethylammonium halides in which the alkyl group has from about 16 to 22 carbon atoms, for example hexadecyltrimethylammonium chloride, cetyltrimethylammonium chloride, behenyltrimethylammonium chloride, dicetyldimethylbenzylammonium chloride, stearyldimethylbenzylammonium chloride, distearyldimethylammonium chloride, cetylpyridinium chloride and their other corresponding halide salts and hydroxides. Preferred cationic surfactants are cetyltrimethylammonium chloride (CTAC) and cetyltrimethylammonium bromide (CTAB). CTAB 99% from Fluka, CTAC 50% (Arquad 16-50, Akzo). Preferably, cationic surfactants are used at 2-10% with CTAC and CTAB being the preferred cationic surfactants. Additionally, when mono-alkyl substituted cationic surfactants are used, it is preferred to also employ cholesterol wherein the ratio of cholesterol to cationic surfactant ranges from 0.1:1.0 to 1.0:1.0, more preferably from 0.5:1.0 to 1.5:1.0, and most preferably 0.7:1.0 to 1.25:1.0.

Specific anionic surfactants are C16-C20 alkyl sulfonates, alkyl ether sulfonates, alkylaryl sulfonates, alkanoyl isethionates, alkyl succinates, alkyl sulfosuccinates, N-alkoyl sarcosinates, alkyl phosphates, alkyl ether phosphates, alkyl ether carboxylates, and alpha-olefin sulfonates, especially their sodium, magnesium, ammonium and triethanolamine salts.

The stabilizing liquid crystals may also be formed from lipid surfactants including either phospholipids, i.e., based on glycerol and sphingosine, or glycolipid, i.e. based on sphingosine. Phospholipids are preferred with phosphatidyl choline (lecithin) being the preferred phospholipid. Of the alcohol moieties which comprise the phosphoglycerides, serine, choline and ethanolamine are particularly preferred, and of the fatty chains, those having a chain length of C14 to C24 are preferred. The fatty acid chains may be branched or unbranched, saturated or unsaturated, and palmitic, myristic, oleic, stearic, arachidonic, linolenic, linoleic and arachidic acids are particularly preferred.

Preferred surfactants for the formation of liquid crystals in the aqueous continuous phase are of the nonionic type and include C16-20 fatty alcohols, C16-20 fatty acids and C11-20 fatty alcohol ethoxylates with 1 to 30 ethylene oxide groups. Specific examples include cetearyl alcohol, cetyl alcohol, stearyl alcohol, oleic acid, arachidyl alcohol, oleyl alcohol, ceteareth ethoxylates with between 10 and 30 ethylene oxide groups, ceteth ethoxylates with between 10 to 30 ethylene oxide groups, steareth ethoxylates with between 10 and 30 ethoxylates, pareth ethoxylates with between 2 and 15 ethoxylates, and combinations thereof. In one preferred embodiment, C 16-20 fatty alcohols and C16-20 fatty acids are used in combination with C16-20 fatty alcohol ethoxylates at a ratio of between 10:1 to 0.5:1, more preferably between 6:1 and 1:1, and most preferably between 5:1 and 1.5:1.

In another preferred embodiment, C16-20 unsaturated fatty alcohols and C16-20 unsaturated fatty acids are used in combination with C16-20 fatty alcohol ethoxylates and C11-15 fatty alcohol ethoxylates.

The presence of liquid crystalline bi-layers, neat phase, can be detected with the use of a polarizing microscope as is taught in "The Microscopy of the Liquid Crystalline Neat and Middle Phases of Soaps and Synthetic Detergents", by F. B. Rosevear in The Journal of the American Oil Chemists' Society, Vol. 31, (December 1954).

<u>**1.2 - The Solvent System:**</u>

The lotions herein comprise a solvent system, in addition to water. As used herein, the term solvent system refers to the system comprising all the solvents in the composition with the exception of water. The solvent system herein has the main function of keeping the surfactant system solubilised to a sufficient degree as to ensure that a thin enough liquid is obtained for the lotion. The solvent system herein typically represents from 5% to 30% by weight of the composition, preferably 15% to 27%. As discussed above, it has been found that sensory irritation is further reduced if the solvent system which is used has a weighted average ClogP of less than 0.0.

The ClogP scale is a convenient means to characterise the degree of hydrophobicity or hydrophilicity of a compound, and is correlated with its octanol/water partitioning coefficient P. The octanol/water partitioning coefficient of a compound is the ratio between its equilibrium concentration in octanol and in water. A compound with a greater partitioning coefficient P is more hydrophobic. Conversely, a compound with a smaller partitioning coefficient P is more hydrophilic. Since the partitioning coefficients of compounds normally have high values, they are more conveniently given in the form of their logarithm to the base 10, logP.

The logP values are most conveniently calculated by the "CLOG P" program, via STN. This database uses experimental and calculated property information available from REGISTRY. There are two sources of experimental properties in the REGISTRY file: Chemical Abstracts Service (CAS) and the SPRESI database produced by ZIC/VINITI

and offered by Infochem. Values for the calculated physical properties in the REGISTRY file were determined using CAS connection tables and software developed by ACD/Labs, Toronto, Canada. Information on the algorithms used to calculate the property information is available on their web site at http://www.acdlabs.com/.

The solvent system herein is preferably characterised by its weighted average ClogP, i.e., the weighted average ClogP of all its constituents. Where the weighted average ClogP can be calculated as follows:

$$\text{Weighted average ClogP} =$$

$$\frac{\text{(amount of solvent A x ClogP of solvent A) + (amount of solvent B x ClogP of solvent B)} \\ \text{+ (amount of solvent C x ClogP of solvent C) + etc}}{\text{amount of solvent A + amount of solvent B + amount of solvent C .......... etc}}$$

This does not include water as a solvent The solvent system herein has a weighted average ClogP of less than 0.0, preferably of less than -0.1 and more preferably of less than -0.3.

Suitable solvents for use in the solvent system herein include, but are not limited to, amides, esters, ethers, ketones, cyclic amides, cyclic esters, cyclic ketones, cyclic ethers, and mixtures thereof. Nonlimiting examples of such solvents include ethyl formate, dimethyl isosorbide, acetylacetone, 2-butanone, acetone, methyl acetate, ethyl acetate, propyl acetate, ethoxyethanol, dipropylene glycol monomethyl ether, butyl lactate, t-butyl alcohol, phenyl acetate, 2-propoxyethanol, isopropoxyethanol, methoxypropanol, isopropyl lactate, hexyl alcohol, butoxyethanol, tripropylene glycol (PPG-3), triacetin, methoxyethanol, isopropyl alcohol, PEG-8, methyl lactate, PEG-6, PEG-5, PEG-4, N-methylpyrrolidone, propyl alcohol, dipropylene glycol (PPG-2), acetonitrile, phenoxyethanol, triethylene glycol, hexylene glycol, ethyl alcohol, □-butyrolactone, butylene glycol, propylene carbonate, dimethyl sulfoxide, diethylene glycol, ethoxydiglycol, propylene glycol, pyrrolidone, pyrrolidone-2, methyl alcohol, ethylene carbonate, ethylene glycol, acetamide, glycerin, butyl carbitol, 1,3-dioxolane, dimethoxymethane, 1,2-hexanediol, dipropylene glycol butyl ether, dipropylene glycol t-butyl ether, propionaldehyde, diethoxymethane and glycerol formal.

Preferred solvents for use in the solvent system herein include lower alkanols, $C_2$-$C_6$ polyols, glycol mono-lower alkyl ethers, diglycol mono-lower-alkyl ethers, and N-lower alkylpyrrolidones. The term "lower" refers to the number of carbon atoms being 3 or less. Specific examples include lower alcanols such as Ethanol, Isopropyl alcohol, lower polyols such as ethylene glycol, propyleneglycol, 1,3-butanediol, diethyleneglycol, glycerine; glycol monoethers such as 2-methoxyethanol and 2-ethoxyethanol; diglycol mono-lower alkyl ethers such as methoxydiglycol, ethoxydiglycol and N-lower-alkylpyrrolidones such as N-methylpyrrolidone and N-ethylpyrrolidone.

The above solvents can be used in any combination to give a weighted average Clog P of $< 0$.

## 1.3 - Monoethanolamine level:

The compositions of the present invention also comprise at least one source of alkalizing agent. It is however preferable that the compositions herein should comprise MEA in the range less than 3.5 wt. %, preferably less than 3 wt. %, more preferably less than 1 wt. %, or from 6 wt % to 20 wt %, more preferably from 9 wt % to 15 wt % MEA.

Any other agent known in the art may be used such as alkanolamides other than monoethanolamine, such as, diethanolamine, triethanolamine, monopropanolamine, dipropanolamine, tripropanolamine, 2-amino-2-methyl-1, 3-propanediol, 2-amino-2-methyl-1-propanol, and 2-amino-2-hydroxymethyl-1,3-propanediol and guanidium salts. Particularly preferred alkalizing agents are those which provide a source of ammonium ions. Any source of ammonium ions is suitable for use herein. Preferred sources include ammonium chloride, ammonium sulphate, ammonium nitrate, ammonium phosphate, ammonium acetate, ammonium carbonate, ammonium hydrogen carbonate, ammonium carbamate, ammonium hydroxide, ammonia and mixtures thereof Particularly preferred are ammonium carbonate and ammonia.

The compositions of the present invention may comprise from about 0.1% to about 10% by weight, preferably from about 0.5% to about 5%, most preferably from about 1% to about 3% of an alkalizing agent, preferably ammonium ions.

### pH

The lotion of the present invention has a pH up to and including pH 11.0. Preferably, the lotion of the present invention has a pH from 9 to 11, more preferably from 10.5 to 11.0.

**1.4 Oxidative Dye Precursor:**

The lotion herein can also be formulated as a hair bleaching lotion or as a hair coloring lotion. Such coloring lotions will also comprise an oxidative hair dye precursors or mixtures thereof (also known as primary intermediates) that will deliver a variety of hair colors to the hair. These precursors are activated by the oxidizing agent which is present in the developer described hereinafter and react with further molecules to form a larger colored complex in the hair shaft.

The lotion comprises from between 0% and 10% oxidative dye precursors, preferably between 0.1 and 5%, and more preferably between 0.2 and 3%. The chemical nature of the oxidative dye precursor(s) present in the lotion does not significantly impact the Lamellar Phase Index.

The precursors can be used alone or in combination with other precursors, and one or more can be used in combination with one or more couplers. Couplers (also known as color modifiers or secondary intermediates) are generally colorless molecules that can form colors in the presence of activated precursors, and are used with other precursors or couplers to generate specific color effects or to stabilize the color.

The choice of precursors and couplers will be determined by the color, shade and intensity of coloration that is desired. The precursors and couplers can be used herein, singly or in combination, to provide dyes having a variety of shades ranging from ash blonde to black.

These compounds are well known in the art, and include aromatic diamines, aminophenols and their derivatives (a representative but not exhaustive list of oxidation dye precursor can be found in Sagarin, "Cosmetic Science and Technology", "Interscience, Special Edn. Vol. 2 pages 308 to 310). It is to be understood that the precursors detailed below are only by way of example and are not intended to limit the compositions and processes herein.

Suitable developers for use in the compositions described herein include, but are not limited to p-phenylenediamine derivatives, e.g. benzene-1,4-diamine (commonly known as p-phenylenediamine), 2-methyl-benzene-1,4-diamine, 2-chloro-benzene-1,4-diamine, N-phenyl-benzene-1,4-diamine, N-(2-ethoxyethyl)benzene-1,4-diamine, 2-[(4-aminophenyl)-(2-hydroxy-ethyl)-amino]-ethanol, (commonly known as N,N-bis(2-hydroxyethyl)-p-phenylenediamine) (2,5-diamino-phenyl)-methanol, 1-(2'-Hydroxyethyl)-2,5-diaminobenzene, 2-(2,5-diamino-phenyl)-ethanol, N-(4-aminophenyl)benzene-1,4-diamine, 2,6-dimethyl-benzene-1,4-diamine, 2-isopropylbenzene-1,4-diamine, 1-[(4-aminophenyl)amino]-propan-2-ol, 2-propyl-benzene-1,4-diamine, 1,3-bis[(4-aminophenyl)(2-hydroxyethyl)amino]propan-2-ol, N4,N4,2-trimethylbenzene-1,4-diamine, 2-methoxy-benzene-1,4-diamine, 1-(2,5-diaminophenyl) ethane-1,2-diol, 2,3-dimethyl-benzene-1,4-diamine, N-(4-amino-3-hydroxy-phenyl)-acetamide, 2,6-diethylbenzene-1,4-diamine, 2,5-dimethylbenzene-1,4-diamine, 2-thien-2-ylbenzene-1,4-diamine, 2-thien-3-ylbenzene-1,4-diamine, 2-pyridin-3-ylbenzene-1,4-diamine, 1,1'-biphenyl-2,5-diamine, 2-(methoxymethyl)benzene-1,4-diamine, 2-(aminomethyl)benzene-1,4-diamine, 2-(2,5-diaminophenoxy)ethanol, N-[2-(2,5-diaminophenoxy)ethyl]-acetamide, N,N-dimethylbenzene-1,4-diamine, N,N-diethylbenzene-1,4-diamine, N,N-dipropylbenzene-1,4-diamine, 2-[(4-aminophenyl) (ethyl)amino]ethanol, 2-[(4-amino-3-methyl-phenyl)-(2-hydroxy-ethyl)-amino]-ethanol, N-(2-methoxyethyl)-benzene-1,4-diamine, 3-[(4-aminophenyl)amino]propan-1-ol, 3-[(4-aminophenyl)-amino]propane-1,2-diol, N-{4-[(4-aminophenyl)amino]butyl}benzene-1,4-diamine, and 2-[2-(2-{2-[(2,5-diaminophenyl)-oxy]ethoxy}ethoxy)ethoxy]benzene-1,4-diamine; 1,3-Bis(N(2-Hydroxyethyl)-N-(4-amino-phenyl)amino)-2-propanol; 2,2'-[1,2-Ethanediyl-bis-(oxy-2,1-ethanediyloxy)]-bis-benzene-1,4-diamine; N,N-Bis(2-hydroxyethyl)-p-phenylinediamine; p-aminophenol derivatives such as: 4-amino-phenol (commonly known as p-aminophenol), 4-methylamino-phenol, 4-amino-3-methyl-phenol, 4-amino-2-hydroxymethyl-phenol, 4-amino-2-methyl-phenol, 4-amino-1-hydroxy-2-(2'-hydroxyethylaminomethyl)benzene, 4-amino-2-methoxymethyl-phenol, 5-amino-2-hydroxy-benzoic acid, 1-(5-amino-2-hydroxy-phenyl)-ethane-1,2-diol, 4-amino-2-(2-hydroxy-ethyl)-phenol, 4-amino-3-(hydroxymethyl)phenol, 4-amino-3-fluoro-phenol, 4-amino-2-(aminomethyl)-phenol, 4-amino-2-fluoro-phenol; 1-Hydroxy-2,4-diaminobenzene; 1-(2'-Hydroxyethyloxy)-2,4-diaminobenzene; 2,4-Diamino-5-methylphenetol; o-phenylenediamine derivatives such as: 3,4-Diaminobenzoic acid and salts thereof; o-aminophenol derivatives such as: 2-amino-phenol (commonly known as o-aminophenol), 2,4-diaminophenol, 2-amino-5-methyl-phenol, 2-amino-6-methyl-phenol, N-(4-amino-3-hydroxy-phenyl)-acetamide, and 2-amino-4-methyl-phenol; and heterocyclic derivatives such as: pyrimidine-2,4,5,6-tetramine (commonly known as 2,4,5,6-tetraaminopyridine), 1-methyl-1H-pyrazole-4,5-diamine, 2-(4,3-diamino-1H-pyrazol-1-yl)ethanol, N2,N2-dimethyl-pyridine-2,5-diamine, 2-[(3-amino-6-methoxypyridin-2-yl)amino]ethanol, 6-methoxy-N2-methyl-pyridine-2,3-diamine, 2,5,6-triaminopyrimidin-4(1H)-one, pyridine-2,5-diamine, 1-isopropyl-1H-pyrazole-4,5-diamine, 1-(4-methylbenzyl)-1H-pyrazole-4,5-diamine, 1-(benzyl)-1H-pyrazole-4,5-diamine, 1-(4-chlorobenzyl)-1H-pyrazole-4,5-diamine, pyrazolo[1,5-a]-pyrimidine-3,7-diamine, 5,6,7-trimethylpyrazolo[1,5-a]pyrimidin-3-ylamine hydrochloride, 7-methylpyrazolo[1,5-a]pyrimidin-3-ylamine hydrochloride, 2,5,6,7-teramethyl-pyrazolo[1,5-a]pyrimidin-3-ylamine hydrochloride, 5,7-di-tert-butylpyrazolo[1,5-a]pyrimidin-3-ylamine hydrochloride, 5,7-di-trifluoromethyl-pyrazolo[1,5-a]pyrimidin-3-ylamine hydrochloride, 2-methylpyrazolo[1,5-a]pyrimidin-3,7-diamine hydrochloride; 4-Hydroxy-2,5,6-triaminopyrimidine; 1-(2'hydroxyethyl)-amino-3,4-methylene dioxybenzene; and 1-Hydroxyethyl-4,5-diaminopyrazole sulphate.

Additional developers are selected from the group consisting of N-(3-furylmethyl)benzene-1,4-diamine; N-Thiophen-3-ylmethyl-benzene-1,4-diamine; N-(2-furylmethyl)benzene-1,4-diamine; N-Thiophen-2-ylmethyl-benzene-1,4-diamine; 4-Hydroxy-benzoic acid (2,5-diamino-benzylidene)-hydrazide; 3-(2,5-Diamino-phenyl)-N-ethyl-acrylamide; 2-[3-(3-Amino-phenylamino)-propenyl]-benzene-1,4-diamine; 2-[3-(4-Amino-phenylamino)-propenyl]-benzene-1,4-diamine; 2-(6-Methyl-pyridin-2-yl)-benzene-1,4-diamine; 2-Pyridin-2-yl-benzene-1,4-diamine; 2-[3-(4-Amino-phenylamino)-propenyl]-benzene-1,4-diamine; 2-[3-(3-Amino-phenylamino)-propenyl]-benzene-1,4-diamine; 3-(2,5-Diamino-phenyl)-N-ethyl-acrylamide; 2-Thiazol-2-yl-benzene-1,4-diamine; 4-Hydroxy-benzoic acid (2,5-diamino-benzylidene)-hydrazide; 3'-Fluoro-biphenyl-2,5-diamine; 2-Propenyl-benzene-1,4-diamine; 2'-Chloro-biphenyl-2,5-diamine; N-Thiophen-3-ylmethyl-benzene-1,4-diamine; N-(3-furylmethyl)benzene-1,4-diamine; 4'-Methoxy-biphenyl-2,5-diamine; N-(4-Amino-benzyl)-benzene-1,4-diamine; 2-Methyl-5-[(1-H-pyrrol-2-ylmethyl)-amino]-phenol; 5-[(Furan-2-ylmethyl)-amino]-2-methyl-phenol; 5-Isopropylamino-2-methyl-phenol; Biphenyl-2,4,4'-triamine hydrochloride; 5-(4-Aminophenyl)aminomethyl-benzene-1,3-diamine hydrochloride; 5-Phenylaminomethyl-benzene-1,3-diamine hydrochloride; 2-[4-Amino-2-(3,5-diamino-benzylamino)-phenoxy]-ethanol hydrochloride; 5-(3-Amino-phenyl) aminomethyl-benzene-1,3-diamine hydrochloride; N-(2-Amino-benzyl)-benzene-1,3-diamine hydrochloride; N-Furan-2-ylmethyl-benzene-1,3-diamine hydrochloride; 2-[(3-Amino-phenylamino)-methyl]-phenol hydrochloride; 4-Amino-2-propylaminomethyl-phenol; hydrochloride; N-Benzo[1,3]dioxol-5-ylmethyl-benzene-1,3-diamine hydrochloride; N-[4-Amino-2-(2-hydroxy-ethyl)-2H-pyrazol-3-yl]-3-(5-amino-2-hydroxy-phenyl)-acrylamide; hydrochloride; 4-Amino-2-(isopropylamino-methyl)-phenol; hydrochloride; 4-Thiophen-3-yl-benzene-1,3-diamine; hydrochloride hydrochloride; 5-Phenylaminomethyl-benzene-1,3-diamine hydrochloride; 5-(3-Amino-phenyl)aminomethyl-benzene-1,3-diamine hydrochloride; 4-Thiophen-3-yl-benzene-1,3-diamine; hydrochloride; 2',4'-Diaminobiphenyl-4-ol; hydrochloride; 5-Cyclobutylamino-2-methyl-phenol; 5-Cyclobutylamino-2-methyl-phenol; 4-Amino-2-(pyridin-3-ylaminomethyl)-phenol; 5-(3-Aminophenyl)aminomethyl-benzene-1,3-diamine hydrochloride; 5-Allylaminomethyl-benzene-1,3-diamine hydrochloride; N-(4-Amino-benzyl)-benzene-1,3-diamine hydrochloride; N-Benzyl-benzene-1,3-diamine hydrochloride; 3-[(3-Amino-phenylamino)-metyl]-phenol hydrochloride; N-(4-Methoxy-benzyl)-benzene-1,3-diamine hydrochloride; N-Thiophen-2-ylmethyl-benzene-1,3-diamine hydrochloride; 4-Amino-2-[(2-hydroxy-5-nitro-phenylamino)-methyl]-phenol; hydrochloride; 2',4'-Diamino-biphenyl-4-ol hydrochloride; Biphenyl-2,4,4'-triamine; 5-(4-Amino-phenyl) aminomethyl-benzene-1,3-diamine hydrochloride; 2-[4-Amino-2-(3,5-diamino-benzylamino)-phenoxy]-ethanol hydrochloride; 5-Allylaminomethyl-benzene-1,3-diamine hydrochloride; 5-(3-Aminophenyl)aminomethyl-benzene-1,3-diamine hydrochloride; N-(4-Amino-benzyl)-benzene-1,3-diamine hydrochloride; N-Benzyl-benzene-1,3-diamine hydrochloride; 3-[(3-Amino-phenylamino)-methyl]-phenol hydrochloride; N-(2-Amino-benzyl)-benzene-1,3-diamine hydrochloride; N-(4-Methoxy-benzyl)-benzene-1,3-diamine hydrochloride; N-Furan-2-ylmethyl-benzene-1,3-diamine hydrochloride; 2-[(3-Amino-phenylamino)-methyl]-phenol hydrochloride; N-Thiophen-2-ylmethyl-benzene-1,3-diamine hydrochloride; N-Benzo[1,3]dioxol-5-ylmethyl-benzene-1,3-diamine hydrochloride; N-[4-Amino-2-(2-hydroxy-ethyl)-2H-pyrazol-3-yl]-3-(5-amino-2-hydroxy-phenyl)-acrylamide hydrochloride; 4-Amino-2-propylaminomethyl-phenol; hydrochloride; 4-Amino-2-(isopropylamino-methyl)-phenol hydrochloride; 4-Amino-2-[(2-hydroxy-5-nitro-phenylamino)-methyl]-phenol hydrochloride; 2-Methyl-5-[(1-H-pyrrol-2-ylmethyl)-amino]-phenol; 5-[(Furan-2-ylmethyl)-amino]-2-methyl-phenol; 5-Isopropylamino-2-methyl-phenol; 5-Cyclobutylamino-2-methyl-phenol; 4-Amino-2-(pyridin-3-ylaminomethyl)-phenol; and 5-Cyclobutylamino-2-methyl-phenol. Preferred developers include but are not limited to: p-phenylenediamine derivatives such as: 2-methyl-benzene-1,4-diamine; benzene-1,4-diamine; 1-(2,5-diamino-phenyl)-ethanol; 2-(2,5-diamino-phenyl)-ethanol; N-(2-methoxyethyl)benzene-1,4-diamine; 2-[(4-amino-phenyl)-(2-hydroxy-ethyl)-amino]-ethanol; 1-(2,5-diaminophenyl)ethane-1,2-diol; 1-(2'-Hydroxyethyl)-2,5-diaminobenzene; 1,3-Bis(N (2-Hydroxyethyl)-N-(4-aminophenyl)amino)-2-propanol; 2,2'-[1,2-Ethanediyl-bis-(oxy-2,1-ethanediyloxy)]-bis-benzene-1,4-diamine; N,N-Bis(2-hydroxyethyl)-p-phenylinediamine; and mixtures thereof; p-aminophenol derivatives such as: 4-amino-phenol, 4-methylamino-phenol, 4-amino-3-methyl-phenol, 4-amino-2-methoxymethyl-phenol; 1-(5-amino-2-hydroxyphenyl)-ethane-1,2-diol; 1-Hydroxy-2,4-diaminobenzene; 1-(2'-Hydroxyethyloxy)-2,4-diaminobenzene; 4-Amino-2-aminomethylphenol; 2,4-Diamino-5-methylphenetol; 4-Amino-1-hydroxy-2-(2'-hydroxyethylaminomethyl)benzene; 1-methoxy-2-amino-4-(2'hydroxyethylamino)benzene; 5-aminosalicylic acid and salts thereof; and mixtures thereof; o-phenylenediamine derivatives such as: 3,4-Diaminobenzoic acid and salts thereof; o-aminophenol derivatives such as: 2-amino-phenol, 2-amino-5-methyl-phenol, 2-amino-6-methyl-phenol, N-(4-amino-3-hydroxy-phenyl)-acetamide; 2-amino-4-methylphenol; and mixtures thereof; and heterocyclic derivatives such as: pyrimidine-2,4,5,6-tetramine; 1-methyl-1H-pyrazole-4,5-diamine; 2-(4,5-diamino-1H-pyrazol-1-yl)ethanol; 1-(4-methylbenzyl)-1H-pyrazole-4,5-diamine; 1-(benzyl)-1H-pyrazole-4,5-diamine; N2,N2-dimethyl-pyridine-2,5-diamine; 4-Hydroxy-2,5,6-triaminopyrimidine; 1-(2'hydroxyethyl)-amino-3,4-methylene dioxybenzene; and 1-Hydroxyethyl-4,5-diaminopyrazole sulphate; and mixtures thereof.

More preferred developers include: 2-methyl-benzene-1,4-diamine; benzene-1,4-diamine; N,N-Bis(2-hydroxyethyl)-p-phenylenediamine; 4-amino-phenol; 4-methylamino-phenol; 4-amino-3-methyl-phenol; 1-Hydroxy-2,4-diaminobenzene; 2-amino-phenol; 2-amino-5-methyl-phenol; 2-amino-6-methyl-phenol; 1-methyl-1H-pyrazole-4,5-diamine; 1-Hydroxyethyl-4,5-diaminopyrazole sulphate; 2-(4,5-diamino-1H-pyrazol-1-yl)ethanol; and mixtures thereof.

Suitable couplers for use in the compositions described herein include, but are not limited to: phenols, resorcinol and naphthol derivatives such as: naphthalene-1,7-diol, benzene-1,3-diol, 4-chlorobenzene-1,3-diol, naphthalen-1-ol, 2-methyl-naphthalen-1-ol, naphthalene-1,5-diol, naphthalene-2,7-diol, benzene-1,4-diol, 2-methyl-benzene-1,3-diol, 7-amino-4-hydroxy-naphthalene-2-sulfonic acid, 2-isopropyl-5-methylphenol, 1,2,3,4-tetrahydro-naphthalene-1,5-diol, 2-chloro-benzene-1,3-diol, 4-hydroxy-naphthalene-1-sulfonic acid, benzene-1,2,3-triol, naphthalene-2,3-diol, 5-dichloro-2-methylbenzene-1,3-diol, 4,6-dichlorobenzene-1,3-diol, 2,3-dihydroxy-[1,4]napbthoquinone; and 1-Acetoxy-2-methylnaphthalene; m-phenylenediamines such as: 2,4-diaminophenol, benzene-1,3-diamine, 2-(2,4-diamino-phenoxy)-ethanol, 2-[(3-amino-phenyl)-(2-hydroxy-ethyl)-amino]-ethanol, 2-mehyl-benzene-1,3-diamine, 2-[[2-(2,4-diamino-phenoxy)-ethyl]-(2-hydroxy-ethyl)-amino]-ethanol, 4-{3-[(2,4-diaminophenyl)oxy]propoxy}benzene-1,3-diamine, 2-(2,4-diamino-phenyl)-ethanol, 2-(3-amino-4-methoxy-phenylamino)-ethanol, 4-(2-amino-ethoxy)-benzene-1,3-diamine, (2,4-diamino-phenoxy)-acetic acid, 2-[2,4-diamino-5-(2-hydroxy-ethoxy)-phenoxy]-ethanol, 4-ethoxy-6-methyl-benzene-1,3-diamine, 2-(2,4-diamino-5-methyl-phenoxy)-ethanol, 4,6-dimethoxy-benzene-1,3-diamine, 2-[3-(2-hydroxy-ethylamino)-2-methyl-phenylamino]-ethanol, 3-(2,4-diamino-phenoxy)-propan-1-ol, N-[3-(dimethylamino)phenyl]urea, 4-methoxy-6-methylbenzene-1,3-diamine, 4-fluoro-6-methylbenzene-1,3-diamine, 2-({3-[(2-hydroxyethyl)amino]-4,6-dimethoxyphenyl}-amino)ethanol, 3-(2,4-diaminophenoxy)-propane-1,2-diol, 2-[2-amino-4-(methylamino)-phenoxy]ethanol, 2-[(5-amino-2-ethoxy-phenyl)-(2-hydroxyethyl)-amino]-ethanol, 2-[(3-aminophenyl)amino]ethanol, 2,4-Diamino-5-(2'-hydroxyethyloxy)toluene; N,N-Dimethyl-3-ureidoaniline; N-(2-aminoethyl)benzene-1,3-diamine, 4-{[(2,4-diamino-phenyl)oxy]methoxy}-benzene-1,3-diamine, 1-methyl-2,6-bis (2-hydroxyethylamino)benzene; and 2,4-dimethoxybenaene-1,3-diamine; m-aminophenols such as: 3-amino-phenol, 2-(3-hydroxy-4-methyl-phenylamino)-acetamide, 2-(3-hydroxy-phenylamino)-acetamide, 5-amino-2-methyl-phenol, 5-(2-hydroxy-ethylamino)-2-methyl-phenol, 5-amino-2,4-dichloro-phenol, 3-amino-2-methyl-phenol, 3-amino-2-chloro-6-methyl-phenol, 5-amino-2-(2-hydroxy-ethoxy)-phenol, 2-chloro-5-(2,2,2-trifluoro-ethylamino)-phenol, 5-amino-4-chloro-2-methyl-phenol, 3-cyclopentylamino-phenol, 5-[(2-hydroxyethyl)amino]-4-methoxy-2-methylphenol, 5-amino-4-methoxy-2-methylphenol, 3-(dimethylamino)phenol, 3-(diethylamino)phenol, 5-amino-4-fluoro-2-methylphenol, 5-amino-4-ethoxy-2-methylphenol, 3-amino-2,4-dichloro-phenol, 3-[(2-methoxyethyl)amino]phenol, 3-[(2-hydroxyethyl)amino]phenol, 5-amino-2-ethyl-phenol, 5-amino-2-methoxyphenol, 5-[(3-hydroxy-propyl)amino]-2-methylphenol, 3-[(3-hydroxy-2-methylphenyl)-amino]propane-1,2-diol, 3-[(2-hydroxyethyl)amino]-2-methylphenol; 1-Methyl-2-hydroxy-4-(2'-hydroxyethyl)aminobenzene; 1,3-Bis-(2,4-Diaminophenoxy)propane; 1-Hydroxy-2-methyl-5-amino-6-chlorobenzene; and heterocyclic derivatives such as: 3,4-dihydro-2H-1,4-benzoxazin-6-ol, 4-methyl-2-phenyl-2,4-dihydro-3H-pyrazol-3-one, 6-methoxyquinolin-8-amine, 4-methylpyzidane-2,6-diol, 2,3-dihydro-1,4-benzodioxin-5-ol, 1,3-benzodioxol-5-ol, 2-(1,3-benzodioxol-5-ylamino)ethanol, 3,4-dimethylpyridine-2,6-diol, 5-chloropyridine-2,3-diol, 2,6-dimethoxypyridine-3,5-diamine, 1,3-benzodioxol-5-amine, 2-{[3,5-diamino-6-(2-hydroxy-ethoxy)-pyridin-2-yl]oxy}-ethanol, 1H-indol-4-ol, 5-amino-2,6-dimethoxypyridin-3-ol, 1H-indole-5,6-diol, 1H-indol-7-ol, 1H-indol-5-ol, 1H-indol-6-ol, 6-bromo-1,3-bemodioxol-5-ol, 2-aminopyridin-3-ol, pyridine-2,6-diamine, 3-[(3,5-diaminopyridin-2-yl)oxy]propane-1,2-diol, 5-[(3,5-diaminopyridin-2-yl)oxy]pentane-1,3-diol, 1H-indole-2,3-dione, indoline-5,6-diol, 3,5-dimethoxypyridine-2,6-diamine, 6-methoxypyridine-2,3-diamine; 3,4-dihydro-2H-1,4-benzoxazin-6-amine; 4-hydroxy-N-methylindole, 1H-5-methylpyrazol-5-one, 1-phenyl-3-methylpyrazol-5-one, 2,6-dimethylpyrazolo[1,5-b]-1,2,4-triazole, 2,6-dimethyl[3,2-c]-1,2,4-triazole, 6-methylpyrazolo-[1,5-a]benzimidazole, 2,6-dihydroxypyridine, 2,6-dihydroxy-3,4-dimethylpyridine, 5-methylpyrazolo[5,1-e]-1,2,3-triazole, 5-methyl-6-chloropyrazolo[5,1-e]-1,2,3,-triazole, 5-phenylpyrazolo[5,1-e]-1,2,3-triazole and its addition salts, 1H-2,6-dimethylpyrazolo[1,5-b]-1,2,4-triazole tosylate, 7,8-dicyano-4-methylimidazolo-[3,2-a]imidazole, 2,7-dimethylpyrazolo[1,5-a]pyrimidin-5-one, 2,5-dimethylpyrazolo[1,5-a]pyrimidin-7-one, and 2-methyl-5-methoxymethyl-pyrazolo[1,5-a]pyrimidin-7-one; 6-Hydroxybenzomorpholine; and 3-Amino-2-methylamino-6-methoxypyridine; 1-Phenyl-3-methyl-5-pyrazolone-2,4-dihydro-5,2-phenyl-3H-pyrazole-3-one.

Preferred couplers include but are not limited to: phenol, resorcinol, and naphthol derivatives such as: naphthalene-1,7-diol, benzene-1,3-diol, 4-chlorobenzene-1,3-diol, naphthalen-1-ol, 2-methyl-naphthalen-1-ol, naphthalene-1,5-diol, naphthalene-2,7-diol, benzene-1,4-diol, 2-methyl-benzene-1,3-diol, and 2-isopropyl-5-methylphenol; 1,2,4-Trihydroxybenzene; 1-Acetoxy-2-methylnaphthalene;and mixtures thereof; m-phenylenediamine derivatives such as: benzene-1,3-diamine, 2-(2,4-diamino-phenoxy)-ethanol, 4-{3-[(2,4-diaminophenyl)oxy]propoxy}benzene-1,3-diamine , 2-(3-amino-4-methoxy-phenylamino)-ethanol, 2-[2,4-diamino-5-(2-hydroxy-ethoxy)-phenoxy]-ethanol, and 3-(2,4-diamino-phenoxy)-propan-1-ol; 2,4-Diamino-5-(2'-hydroxyethyloxy)toluene; N,N-Dimethyl-3-ureidoaniline; 2,4-Diamino-5-fluorotoluenesulfatehydrate; 1-methyl-2,6-bis(2-hydroxyethylamino)benzene; and mixtures thereof; m-aminophenol derivatives such as: 3-amino-phenol, 5-amino-2-methyl-phenol, 5-(2-hydroxy-ethylamino)-2-methylphenol, and 3-amino-2-methyl-phenol; I-Methyl-2-hydroxy-4-(2'-hydroxyethyl)aminobenzene; 1-Hydroxy-3-amino-2,4-dichlorobenzene; 1,3-Bis-(2,4-Diaminophenoxy)propane; 1-Hydroxy-2-methyl-5-amino-6-chlorobenzene; 5-Amino-4-chloro-2-methylphenol; and mixtures thereof; and heterocyclic derivatives such as: 3,4-dihydro-2H-1,4-benzoxazin-6-ol, 4-methyl-2-phenyl-2,4-dihydro-3H-pyrazol-3-one, 1,3-benzodioxol-5-ol, 1,3-benzodioxol-5-amine, 1H-indol-4-ol, 1H-indole-5,6-diol, 1H-indol-7-ol, .1H-indol-5-ol, 1H-indol-6-ol, 1H-indole-2,3-dione, pyridine-2,6-diamine, 2-aminopyridin-3-ol, 4-hydroxy-N-methylindole, 1H-5-methylpyrazol-5-one, 1-phenyl-3-methylpyrazol-5-one, 2,6-dimethylpyrazolo[1,5-b]-1,2,4-triazole, 2,6-dimethyl[3,2-c]-1,2,4-triazole, 6-methylpyrazolo-[1,5-a]benzimidazole; 2,6-dihydroxypyridine; 2,6-dihydroxy-3,4-dimethylpyridine; 6-Hydroxybenzomorpholine; 2,6-Dihydroxy-3,4-dimethylpyridine; 3,5-Diamino-2,6-dimethoxypyridine; 3-Amino-2-methylamino-6-methoxypyridine; 1-Phenyl-3-methyl-5-pyrazolone-2,4-dihydro-5,2-phenyl-3H-pyrazole-3-one; and mixtures thereof.
More preferred couplers include: benzene-1,3-diol; 4-chlorobenzene-1,3-diol; 2-methylbenzene-1,3-diol; benzene-1,3-diamine; 3-amino-phenol; 5-amino-2-methyl-phenol; 1-Methyl-2-hydroxy-4-(2'-hydroxyethyl)aminobenzene; 4-methyl-2-phenyl-2,4-dihydro-3H-pyrazol-3-one; 2-aminopyridin-3-ol; 1-phenyl-3-methylpyrazol-5-one; 1-Phenyl-3-methyl-5-pyrazolone-2,4-dihydro-5,2-phenyl-3H-pyrazole-3-one; and mixtures thereof.

Hair dye compositions will generally comprise from about 0.001% to about 10% of dyes. For example compositions providing low intensity dyeing such as natural blond to light brown hair shades generally comprise from about 0.001% to about 5%, preferably from about 0.1% to about 2%, more preferably from about 0.2% to about 1% by weight of dyeing composition of precursors and couplers. Darker shades such as browns and black typically comprise from 0.001% to about 10% by weight, preferably from about 0.05% to about 7% by weight, more preferably form about 1% to about 5% of precursors and couplers.

In the embodiment where the lotion is a bleaching lotion, no oxidative dye is present. Instead the lotion provides only a source of alkaliser which raises and buffers the pH of the developer producing active bleaching species and aiding fiber penetration.

## 2 - The Developer:

The developer comprises a scalp-irritating amount of an oxidising agent in an aqueous matrix. The developer herein also has a viscosity of 1 cps to 5000 cps, preferably 1 cps to 500 cps (all viscosities herein are measured at 6 rpm with a Brookfield DV-11+ viscometer with a T-Bar D spindle at 25°C), and its viscosity prior to mixing with the lotion can be adjusted with the use of conventional thickeners which are resistant in an oxidative environment.

### 2.1 - Oxidizing agent:

The developer in the present invention comprises a scalp-irritating amount of an oxidizing agent. Preferred oxidizing agents for use herein are water-soluble peroxygen oxidizing agents. "Water-soluble" as defined herein means that in standard condition at least 0.1g, preferably 1g, more preferably 10g of said oxidizing agent can be dissolved in 1 liter of deionized water. The oxidizing agents are valuable for the initial solubilisation and decolorisation of the melanin (bleaching) and accelerate the polymerization of the oxidative dye precursors (oxidative dyeing) in the hair shaft.

Preferred water-soluble oxidizing agents are inorganic peroxygen materials capable of yielding hydrogen peroxide in an aqueous solution. Water-soluble peroxygen oxidizing agents are well known in the art and include hydrogen peroxide, inorganic alkali metal peroxides such as sodium periodate and sodium peroxide and organic peroxides such as urea peroxide, melamine peroxide, and inorganic perhydrate salt bleaching compounds, such as the alkali metal salts of perborates, percarbonates, perphosphates, persilicates, persulphates and the like. These inorganic perhydrate salts may be incorporated as monohydrates, tetrahydrates etc. Mixtures of two or more such oxidizing agents can be used if desired. Preferred for use in the compositions according to the present invention are hydrogen peroxide, percarbonate, persulphates and combinations thereof.

Examples of oxidising agents include hydrogen peroxide, peroxyurea, peroxymelamine, sodium perborate, potassium perborate, sodium percarbonate, potassium perborate, sodium percarbonate and potassium percarbonate with hydrogen peroxide being particularly preferred.

According to the present invention, the compositions comprise more than 3% activity, preferably more than 3.5% activity and most preferably more than 4.5% activity of an oxidizing agent.

Another preferred oxidizing agent for use herein is a source of peroxymonocarbonate ions. Preferably such a source is formed in situ from a source of hydrogen peroxide and a hydrogen carbonate ion source. Such an oxidizing agent has been found to be particularly effective at a pH of up to and including 9.5, preferably 7.5 to 9.5 more preferably about pH 9. Moreover, this system is also particularly effective in combination with a source of ammonia or ammonium ions. It has been found that this oxidizing agent can deliver improvements to the desired hair colour results particularly with regard to the delivery of high lift, whilst considerably reducing the odour, skin and scalp irritation and damage to the hair fibres.

Accordingly, any source of these ions may be utilized. Suitable sources for use herein include sodium, potassium, guanidine, arginine, lithium, calcium, magnesium, barium, ammonium salts of carbonate, carbamate and hydrocarbonate ions and mixtures thereof such as sodium carbonate, sodium hydrogen carbonate, potassium carbonate, potassium hydrogen carbonate, guanidine carbonate, guanidine hydrogen carbonate, lithium carbonate, calcium carbonate, magnesium carbonate, barium carbonate, ammonium carbonate, ammonium hydrogen carbonate and mixtures thereof. Percarbonate salts may also be utilized to provide both the source of carbonate ions and oxidizing agent. Preferred sources of carbonate ions, carbamate and hydrocarbonate ions are sodium hydrogen carbonate, potassium hydrogen carbonate, ammonium carbamate, and mixtures thereof.

According to the present invention the compositions comprise from about 0.1% to about 15% by weight, preferably from about 1% to about 10% by weight, and most preferably from about 1% to about 8% by weight of a hydrogencarbonate ion and from about 0.1% to about 10% by weight, preferably from about 1% to about 7% by weight, and most preferably from about 2% to about 5% by weight of a source of hydrogen peroxide.

**2.2 - Source of alkalizing agent :**

According to the present invention the composition also comprises at least one source of alkalizing agent, preferably a source of ammonium ions and or ammonia. Any agent known in the art may be used such as alkanolamides for example monoethanolamine, diethanolamine, triethanolamine, monopropanolamine, dipropanolamine, tripropanolamine, 2-amino-2-methyl-1, 3-propanediol, 2-amino-2-methyl-1-propanol, and 2-amino-2-hydroxymethyl-1,3-propanediol and guanidium salts. Particularly, preferred alkalizing agents are those which provide a source of ammonium ions. Any source of ammonium ions is suitable for use herein. Preferred sources include ammonium chloride, ammonium sulphate, ammonium nitrate, ammonium phosphate, ammonium acetate, ammonium carbonate, ammonium hydrogen carbonate, ammonium carbamate, ammonium hydroxide, percarbonate salts, ammonia and mixtures thereof. Particularly preferred are ammonium carbonate, ammonium carbamate, ammonia and mixtures thereof

The compositions of the present invention may comprise from about 0.1% to about 10% by weight, preferably from about 0.5% to about 5%, most preferably from about 1% to about 3% of an alkalizing agent, preferably ammonium ions.

**23 - PH:**

The developer of the present invention has a pH up to and including pH 3.5. Preferably, the developer of the present invention have a pH of from 2.4 to 3.1.

**2.4 - Additional components:**

The compositions of the present invention may comprise additional ingredients which include, but are not limited to, enzymes, radical scavengers, conditioning agents, carriers, antioxidants (such as erythorbic acid, sodium metabisulphite, tocopherol acetate, tocopherol), stabilizers, chelants, perming actives, perfume, reducing agents (thiolactic acid), hair swelling agents and/or polymers.

In addition other miscellaneous surfactants can be employed within the surfactant system provided that a Lamellar Phase Index of greater than 80% is achieved within the lotion formulation environment. These include numerous varieties of non-ionic surfactants, anionic surfactants, cationic surfactants, amphoteric surfactants, and zwitterionic surfactants.

Miscellaneous nonionic surfactants that may be used include, but are not limited to, trideceth-6, ceteth-10, laureth-9, octoxynol-9, nonoxynol-12, a poloxamer, trideceth-12, oleth-20 and polysorbate-20. Numerous other nonionic surfactants are listed in McCutcheon's Emulsifiers and Detergents, North American and International Editions, MC Publishing Co., Glen Rock NJ, pages 235-246 (1993).

Miscellaneous cationic surfactants include, but are not limited to, lauryl dimethyl amine oxide, stearyl dimethyl amine oxide, cocoamidopropyl dimethylamine oxide, stearamidopropyl dimethylamine, C14-20 isoalkylamidopropylethyldimonium ethosulfate, C18-22 isoalkylamidopropylethyldimonium ethosulfate, cocamidopropyldimonium hydroxypropylamino hydrolyzed animal protein, hydroxyanthraquinoneaminopropyl methyl morpholinium methosulfate, oleamidopropyl ethyldimonium ethosulfate, rapeseedamidopropyl benzyldimonium chloride, rapeseedamidopropyl ethyldimonium ethosulfate, ricinoleamidopropyl ethyldimonium ethosulfate, soyamidopropyl benzyldimonium chloride, soyamidopropyl ethyldimonium ethosulfate, stearamidopropalkonium chloride, stearamidopropyl cetearyl dimonium tosylate, and mixtures thereof.

Miscaellaneous anionic surfactants include, but are not limited to, compounds in the classes known as alkyl sulfates, alkyl ether sulfates, alkylamide sulfates, alkylaminde ether sulfates, alkyl ether sulfonates, alkylamide sulfonates, sulfate esters of an alkyl carbonates, alkyl ether carboxylates, fatty acids, soaps, alkyl sulfosuccinates, alkyl ether sulfosuccinates, alkylamide sulfosuccinates, sarcosinates, alkyl phosphates, octoxynol or nonoxynol phosphates, taurates, fatty taurides, sulfated monoglycerides, fatty acid amido polyoxyethylene sulfates, isethionates, and mixtures thereof. Usually, the anionic surfactant is present in the composition as a neutralized salt in the form of a sodium, potassium, lithium, ammonium, alkylammonium or hydroxyalkylammonium salt, wherein the alkyl moiety includes from one to about three carbon atoms.

Miscellaneous amphoteric surfactants include, but are not limited to, cocoamidopropyl dimethyl betaine, cocoamidopropyl hydroxysultaine, cocodimethyl betaine, coco imidazoline dicarboxylate, coco imidazoline monocarboxylate, cocobetaine, lapyrium chloride, lauryl sultaine, decyl betaine, oleamidopropyl betaine, tallowamidopropyl betaine,

sodium caproamphacetate, sodium lauroamphoacetate, sodium cocamphopropylsulfonate, numerous other alkylamido alkylamines and betaines listed in the CTFA Cosmetic Ingredient Handbook, 1 st Ed., 1988 at pages 9, 10, 15 and 16.

Many additional miscellaneous nonionic, cationic, anionic, and amphoteric surfactants that may be used are listed in McCUTCHEON'S EMULSIFIERS AND DETERGENTS, 1993 ANNUAL, published by McCutcheon Division, MC Publication Co., Glen Rock, NJ, and in the CTFA Handbook.

According to the present invention the compositions may further comprise a source of radical scavenger. As used herein the term radical scavenger refers to a species that can react with a reactive radical, preferably carbonate radicals, to convert the reactive radical by a series of fast reactions to a less reactive species.

Suitable radical scavengers for use herein include compounds according to the general formula:

$$(I): R^1\text{-}Y\text{-}C(H)(R^3)\text{-}R^4\text{-}(C(H)(R^5)\text{-}Y\text{-}R^6)_n$$

wherein Y is $NR^2$, O, or S, preferably $NR^2$, n is 0 to 2, and wherein $R^4$ is monovalent or divalent and is selected from: (a) substituted or unsubstituted, straight or branched, alkyl, mono- or poly-unsaturated alkyl, heteroalkyl, aliphatic, heteroaliphatic, or heteroolefinic systems, (b) substituted or unsubstituted, mono- or poly-cyclic aliphatic, aryl, or heterocyclic systems, or (c) substituted or unsubstituted, mono-, poly-, or per-fluoro alkyl systems; the systems of (a), (b) and (c) comprising from 1 to 12 carbon atoms and 0 to 5 heteroatoms selected from O, S, N, P, and Si; and wherein $R^4$ can be connected to $R^3$ or $R^5$ to create a 5, 6 or 7 membered ring; and wherein $R^1$, $R^2$, $R^3$, $R^5$, and $R^6$ are monovalent and are selected independently from: (a), (b) and (c) described herein above, or H.

Preferably, $R^4$ is selected from: (a) substituted or unsubstituted, straight or branched, alkyl, heteroalkyl, aliphatic, heteroaliphatic, or heteroolefinic systems, (b) substituted or unsubstituted, mono- or poly-cyclic aliphatic, aryl, or heterocyclic systems, or (c) substituted or unsubstituted, mono-, poly-, or per-fluoro alkyl systems; more preferably $R^4$ is selected from (a) substituted or unsubstituted, straight or branched, alkyl, heteroalkyl, aliphatic, or heteroaliphatic systems, (b) substituted or unsubstituted, aryl, or heterocyclic systems, or (c) substituted or unsubstituted, mono-, poly-, or per-fluoro alkyl systems; more preferably substituted or unsubstituted, straight or branched, alkyl, or heteroalkyl systems.

Preferably, the $R^4$ systems of (a), (b), and (c), described herein above, comprise from 1 to 8 carbon atoms, preferably from 1 to 6, more preferably from 1 to 4 carbon atoms and from 0 to 3 heteroatoms; preferably from 0 to 2 heteroatoms; most preferably from 0 to 1 heteroatoms. Where the systems contain heteroatoms, preferably they contain 1 heteroatom. Preferred heteroatoms include O, S, and N; more preferred are O, and N; and most preferred is O.

Preferably, $R^1$, $R^2$, $R^3$, $R^5$, and $R^6$ are selected independently from any of the systems defined for $R^4$ above, and H.

In alternative embodiments, any of $R^1$, $R^2$, $R^3$, $R^4$, $R^5$, and $R^6$ groups are substituted. Preferably, the substituent(s) is selected from: (a) the group of C-linked monovalent substituents consisting of: (i) substituted or unsubstituted, straight or branched, alkyl, mono- or poly-unsaturated alkyl, heteroalkyl, aliphatic, heteroaliphatic, or heteroolefinic systems, (ii) substituted or unsubstituted, mono- or poly-cyclic aliphatic, aryl, or heterocyclic systems, or (iii) substituted or unsubstituted, mono-, poly-, or per-fluoro alkyl systems; said systems of (i), (ii) and (iii) comprising from 1 to 10 carbon atoms and 0 to 5 heteroatoms selected from O, S, N, P, and Si; (b) the group of S-linked monovalent substituents consisting of $SA^1$, SCN, $SO_2A^1$, $SO_3A^1$, $SSA^1$, $SOA^1$, $SO_2NA^1A^2$, $SNA^1A^2$, and $SONA^1A^2$; (c) the group of O-linked monovalent substituents consisting of $OA^1$, OCN and $ONA^1A^2$; (d) the group of N-linked monovalent substituents consisting of $NA^1A^2$, $(NA^1A^2A^3)^+$, NC, $NA^1OA^2$, $NA^1SA^2$, NCO, NCS, $NO_2$, $N{=}NA^1$, $N{=}NOA^1$, $NA^1CN$, $NA^1NA^2A^3$; (e) the group of monovalent substituents consisting of $COOA^1$, $CON_3$, $CONA^1{}_2$, $CONA^1COA^2$, $C(=NA^1)NA^1A^2$, CHO, CHS, CN, NC, and X; and (f) the group consisting fluoroalkyl monovalent substituents consisting of mono-, poly-, or per-fluoro alkyl systems comprising from 1 to 12 carbon atoms and 0 to 4 heteroatoms.

For the groups (b) to (e), described above, $A^1$, $A^2$, and $A^3$ are monovalent and are independently selected from: (1) H, (2) substituted or unsubstituted, straight or branched, alkyl, mono- or poly-unsaturated alkyl, heteroalkyl, aliphatic, heteroaliphatic, or heteroolefinic systems, (3) substituted or unsubstituted, mono- or poly-cyclic aliphatic, aryl, or heterocyclic systems, or (4) substituted or unsubstituted, mono-, poly-, or per-fluoro alkyl systems; said systems of (2), (3) and (4) comprising from 1 to 10 carbon atoms and 0 to 5 heteroatoms selected from O, S, N, P, and Si; and wherein X is a halogen selected from the group consisting of F, Cl, Br, and I.

Preferred substituents for use herein include those having a Hammett Sigma Para ($\sigma_p$) Value from -0.65 to +0.75, preferably from -0.4 to +0.5. Hammett Sigma Values are described in Advanced Organic Chemistry - Reactions, Mechanisms and Structure (Jerry March, 5th ed. (2001) at pages 368-375).

Alternative suitable radical scavengers for use herein are compounds according to the general formula (II) :

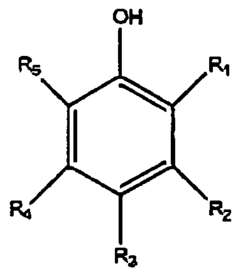

(II)

wherein $R_1$, $R_2$, $R_3$, $R_4$, and $R_5$ are each independently selected from H, $COO^-M^+$, Cl, Br, $SO_3^-M^+$, $NO_2$, $OCH_3$, OH or a $C^1$ to $C^{10}$ primary or secondary alkyl and M is either H or alkali metal. Preferably, the above-described radical scavengers have a pKa of more than 8.5 to ensure protonation of the hydroxy goup.

Other suitable radical scavengers for use herein include those selected from group (III) benzylamine, imidazole, di-tert-butylhydroxytoluene, hydroquinone, guanine, pyrazine, piperidine, morpholine, methylmorpholine, 2methyoxyethylamine, and mixtures thereof.

Preferred radical scavengers according to the present invention are selected from the classes of alkanolamines, amino sugars, amino acids, esters of amino acids and mixtures thereof. Particularly preferred compounds are: monoethanolamine, 3-amino-1-propanol, 4-amino-1-butanol,5-amino-1-pentanol, 1-amino-2-propanol, 1-amino-2-butanol, 1-amino-2-pentanol, 1-amino-3-pentanol, 1-amino-4-pentanol, 3-amino-2-methylpropan-1-ol, 1-amino-2-methylpropan-2-ol, 3-aminopropane-1,2-diol, glucosamine, N-acetylglucosamine, glycine, arginine, lysine, proline, glutamine, histidine, sarcosine, serine, glutamic acid, tryptophan, and mixtures thereof, and the salts such as the potassium, sodium and ammonium salts thereof and mixtures thereof. Especially preferred compounds are glycine, sarcosine, lysine, serine, 2 methoxyethylamine, glucosamine, glutamic acid, morpholine, piperdine, ethylamine, 3 amino-1-propanol and mixtures thereof

The radical scavengers according to the present invention preferably have a molecular weight of less than about 500, preferably less than about 300, more preferably less than about 250 in order to facilitate penetration of the radical scavenger into the hair fibre. The compositions of the present invention preferably comprise from about 0.1% to about 10% by weight, preferably from about 1% to about 7% by weight of radical scavenger. The radical scavenger is also preferably selected such that it is not an identical species as the alkalizing agent. According to one embodiment of the present invention the radical scavenger may be formed insitu in the hair dyeing compositions prior to application to the hair fibres.

In another embodiment of the present invention, there is provided a process of coloring hair with the system of the invention, wherein said lotion and said developer are mixed together so as to create a thickened coloring composition, and said thickened coloring composition is applied to hair, left to act, then rinsed off. The thickened coloring composition, as used herein, has a viscosity of from 7,000 to 25,000 cps, preferably 8,000 cps to 15,000 cps, most preferably 10,000 cps to 12,000 cps.

In addition, the compositions of the present invention may comprise or may be used in combination with a composition comprising a conditioning agent. Conditioning agents suitable for use herein are selected from silicone materials, amino silicones, fatty alcohols, polymeric resins, polyol carboxylic acid esters, cationic polymers, cationic surfactants, insoluble oils and oil derived materials and mixtures thereof. Additional materials include mineral oils and other oils such as glycerin and sorbitol.

The conditioning agent will generally be used at levels of from about 0.05% to about 20% by weight of the composition, preferably of from about 0.1% to about 15%, more preferably of from about 0.2% to about 10%, even more preferably of from about 0.2% to about 2%.

According to the present invention the compositions may comprise chelants. Chelants are well known in the art and refer to a molecule or a mixture of different molecules each capable of forming a chelate with a metal ion. Chelants are well known in the art and a non-exhaustive list thereof can be found in AE Martell & RM Smith, Critical Stability Constants, Vol. 1, Plenum Press, New York & London (1974) and AE Martell & RD Hancock, Metal Complexes in Aqueous Solution, Plenum Press, New York & London (1996) both incorporated herein by reference.

Examples of chelants suitable for use herein include EDDS (ethylenediaminedisuceinic acid), carboxylic acids (in particular aminocarboxylic acids), phosphonic acids (in particular aminophosphonic acids) and polyphosphoric acids (in particular linear polyphosphoric acids), their salts and derivatives.

Chelants may be incorporated into the composition of the present invention as stabilizers and or preservatives. In addition it has also been found that chelants provide hair fibre damage benefits and thus they be utilized in order to further improve the hair damage profile of the present invention. Levels of chelants in the present invention may be as

low as about 0.1%, preferably at least about about 0.25%, more preferably about 0.5% for the most effective chelants such as diamine-N,N'-dipolyacid and monoamine monoamide-N,N'-dipolyacid chelants (for example EDDS). Less effective chelants will be more preferably used at levels of at least about 1%, even more preferably above about 2% by weight of the composition, depending of the efficiency of the chelant. Levels as high as about 10% can be used, but above this level significant formulation issues may arise.

**EXAMPLES**

The following examples illustrate the invention.

The lotions shown below are prepared by mixing the separate components. When these four lotions are separately mixed with a commercially available developer (30 volume Nice 'N Easy ® Developer, which contains 4.5% hydrogen peroxide), four differing gelled hair colouring compositions are prepared which color human hair when applied thereto and thoroughly incorporated in the hair by finger manipulation.

Lotions:

| Raw Material | 1 | 2 | 3 | 4 |
|---|---|---|---|---|
| | | | | |
| Soytrimonium Chloride | 7.3 | 7.3 | 7.3 | 7.3 |
| Propylene Glycol | 0 | 19.4 | 9.6 | 9.6 |
| Cocamidopropyl Betaine (30%) | 3.3 | 3.3 | 3.3 | 3.3 |
| Ethoxydiglycol | 0 | 0 | 3.3 | 3.3 |
| Monoethanolamine LCI | 3.3 | 3.3 | 3.3 | 0 |
| Oleth 5 | 2.9 | 2.9 | 2.9 | 2.9 |
| Oleth 2 | 1.5 | 1.5 | 1.5 | 1.5 |
| Oleic Acid | 2.6 | 2.6 | 2.6 | 2.6 |
| C11-C15 Pareth-9 | 0.9 | 0.9 | 0.9 | 0.9 |
| C12-C15 Pareth-3 | 0.5 | 0.5 | 0.5 | 0.5 |
| Hexylene Glycol | 19.4 | 0 | 0 | 0 |
| EDDS | 6.7 | 6.7 | 6.7 | 6.7 |
| Fragrance AM-2672 | 1.3 | 1.3 | 1.3 | 1.3 |
| Erythorbic Acid | 0.4 | 0.4 | 0.4 | 0.4 |
| Citric Acid Anhydrous | 0.4 | 0.4 | 0.4 | 0.4 |
| Sodium Sulphite | 0.1 | 0.1 | 0.1 | 0.1 |
| EDTA | 0.05 | 0.05 | 0.05 | 0.05 |
| Isopropyl Alcohol (IPA) | 0 | 0 | 1.5 | 1.5 |
| Ammonium Hydroxide | 7.0 | 7.0 | 7.0 | 8.0 |
| Butoxydiglycol<br>Oxidative Dye Precursors<br>Water | 0<br>0.33<br>42.02 | 0<br>0.33<br>42.02 | 4.5<br>0.33<br>42.52 | 4.5<br>0.33<br>44.82 |
| Lamellar Phase Index | 25% | 100% | 84.2% | 88.4% |
| Sensory score in Clinical | High | Low | Medium | Moderate |

A clinical test evaluates the sensory effects of hair colourant products placed on the human forearm. Pannelists then rate the products using an established scale. A high score means a high sensory irritation. Composition 1 is not

according to the invention. It has a Lamellar Phase Index of only 25% and gets a high score in the clinical test. Composition 2 is also not according to the invention. It has a Lamellar Phase Index of 100, obtains a low score in the test, but is unstable. Compositions 3 and 4 are according to the invention, they get acceptable scores in the test and are stable.

Further example formulations of the present invention are given below:-

| Raw Material | 5 | 6 |
|---|---|---|
| | | |
| Soytrimonium Chloride | 7.3 | 7.3 |
| Propylene Glycol | 9.6 | 9.6 |
| Cocamidopropyl Betaine (30%) | 3.3 | 3.3 |
| Ethoxydiglycol | 3.3 | 3.3 |
| Oleth 5 | 2.9 | 2.9 |
| Oleth 2 | 1.5 | 1.5 |
| Oleic Acid | 2.6 | 2.6 |
| C11-C15 Pareth-9 | 0.9 | 0.9 |
| C12-C15 Pareth-3 | 0.5 | 0.5 |
| Hexylene Glycol | 0 | 0 |
| Fragrance AM-2672 | 1.3 | 1.3 |
| Erythorbic Acid | 0.4 | 0.4 |
| Citric Acid Anhydrous | 0.4 | 0.4 |
| Sodium Sulphite | 0.1 | 0.1 |
| EDTA | 0.05 | 0.05 |
| Isopropyl Alcohol (IPA) | 1.5 | 1.5 |
| Ammonium Carbonate | 5.0 | 5.0 |
| Sodium Glycinate | 2.5 | 0.0 |
| Butoxydiglycol | 4.5 | 4.5 |
| Oxidative Dye Precursors | 0.33 | 0.33 |
| Water | 51.52 | 54.02 |

## Claims

1. A two part system for coloring or bleaching hair which comprises (i) a thin lotion optionally comprising a oxidative dye precursor and comprising a solvent system and a surfactant system to trigger a thickening upon dilution with a developer, and (ii) a developer which comprises a scalp-irritating amount of an oxidising agent, **characterised in that** said lotion has a Lamellar Phase Index of greater than 80% and less than 100%.

2. A system according to claim 1, wherein said lotion has a Lamellar Phase Index of from 80% to 95%.

3. A system according to the preceding claims, wherein said developer comprises hydrogen peroxide as the oxidant.

4. A system according to claim 3, wherein said developer comprises more than 3.0% hydrogen peroxide, preferably more than 3.5% peroxide, more preferably more than 4.5% peroxide.

5. A system according to any of the preceding claims, wherein the solvent system in said lotion is predominantly hydrophilic.

**6.** A system according to claim 5, wherein said solvent system has a weighted average ClogP of less than 0.

**7.** A system according to any of the preceding claims, wherein said lotion comprises from 5 % to 30% solvent, more preferably from 15 to 27% solvent.

**8.** A system according to any of the preceding claims wherein said lotion comprises less than 3.5 wt. % MEA, preferably less than 3 wt. %, more preferably less than 1 wt. %, or from 6 wt % to 20 wt %, more preferably from 9 wt % to 15 wt % MEA.

**9.** A system according to the preceding claims, wherein said lotion and said developer each have a viscosity comprised between 1 to 5000cPs, and said coloring composition resulting from the mixture of said lotion and said developer has a viscosity of 7,000 and 25,000 cPs.

**10.** A process of coloring or bleaching hair with the system according to any of the preceding claims, wherein said lotion and said developer are mixed together so as to create a thickened coloring or bleaching composition, and said thickened coloring or bleaching composition is applied to hair, left to act, then rinsed off.

European Patent Office

## PARTIAL EUROPEAN SEARCH REPORT

which under Rule 45 of the European Patent Convention shall be considered, for the purposes of subsequent proceedings, as the European search report

Application Number
EP 05 01 1328

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | WO 02/092035 A (CLAIROL INC) 21 November 2002 (2002-11-21) * page 23, line 1 - page 26, line 26; claims 1,4-7; examples * ----- | 1-10 | A61K7/13 A61K7/135 |
| A | WO 99/36047 A (SQUIBB BRISTOL MYERS CO) 22 July 1999 (1999-07-22) * claims * ----- | 1-10 | |

TECHNICAL FIELDS SEARCHED (Int.Cl.7)

A61K

### INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

1

| Place of search | Date of completion of the search | Examiner |
|---|---|---|
| The Hague | 29 August 2005 | Couckuyt, P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

European Patent Office

INCOMPLETE SEARCH SHEET C

Application Number
EP 05 01 1328

Claim(s) searched completely:
3-10

Claim(s) searched incompletely:
1

Claim(s) not searched:
2

Reason for the limitation of the search:

Present claims 1 and 2 relate to a two part system for coloring and bleaching and corresponding process defined (inter alia) by reference to the following parameter: 'lotion has a Lamellar Phase Index of greater than 80% and less than 100%'.

The use of this parameter in the present context is considered to lead to a lack of clarity within the meaning of Article 84 EPC. It is impossible to compare the parameter the applicant has chosen to employ with what is set out in the prior art, as it is not generally recognized in the international practice. Therefore, a 'lotion has a Lamellar Phase Index of greater than 80% and less than 100%', does not allow to clearly define the composition.
The lack of clarity is such as to render a meaningful complete search impossible. Consequently, the search has been performed without taking the above parameter into account, with due regard to the general idea underlying the application.

## ANNEX TO THE EUROPEAN SEARCH REPORT ON EUROPEAN PATENT APPLICATION NO. EP 05 01 1328

This annex lists the patent family members relating to the patent documents cited in the above-mentioned European search report.
The members are as contained in the European Patent Office EDP file on
The European Patent Office is in no way liable for these particulars which are merely given for the purpose of information.

29-08-2005

| Patent document cited in search report | Publication date | Patent family member(s) | Publication date |
|---|---|---|---|
| WO 02092035 A | 21-11-2002 | US 2003005526 A1 | 09-01-2003 |
| | | BR 0209649 A | 13-07-2004 |
| | | CA 2446056 A1 | 21-11-2002 |
| | | CN 1543333 A | 03-11-2004 |
| | | EP 1387661 A1 | 11-02-2004 |
| | | JP 2004529949 T | 30-09-2004 |
| | | MX PA03009651 A | 29-01-2004 |
| | | WO 02092035 A1 | 21-11-2002 |
| | | US 2002194683 A1 | 26-12-2002 |
| WO 9936047 A | 22-07-1999 | US 6156076 A | 05-12-2000 |
| | | AT 281146 T | 15-11-2004 |
| | | AU 762113 B2 | 19-06-2003 |
| | | AU 1633799 A | 02-08-1999 |
| | | BR 9814008 A | 10-10-2000 |
| | | CA 2317487 A1 | 22-07-1999 |
| | | CN 1285736 A ,C | 28-02-2001 |
| | | DE 69827412 D1 | 09-12-2004 |
| | | EP 1047375 A1 | 02-11-2000 |
| | | JP 2002509099 T | 26-03-2002 |
| | | US 2003140430 A1 | 31-07-2003 |
| | | WO 9936047 A1 | 22-07-1999 |

EPO FORM P0459

For more details about this annex : see Official Journal of the European Patent Office, No. 12/82